Europäisches Patentamt

European Patent Office

Office européen des brevets

(19)

(11) Publication number: **0 007 244**
**B1**

(12) **EUROPEAN PATENT SPECIFICATION**

(45) Date of publication of patent specification: **11.05.83**

(51) Int. Cl.³: **C 12 N 5/02, C 12 P 17/12**

(21) Application number: **79301412.7**

(22) Date of filing: **17.07.79**

(54) **Preparing nicotine by culturing a nicotiana strain.**

(30) Priority: **19.07.78 GB 3038178**

(43) Date of publication of application:
**23.01.80 Bulletin 80/2**

(45) Publication of the grant of the patent:
**11.05.83 Bulletin 83/19**

(84) Designated Contracting States:
**AT BE CH DE FR IT LU NL SE**

(56) References cited:
**FR - A - 2 112 434**
**FR - A - 2 138 121**

**PHYSIOL. PLANT. 38., 1976, pages 19—23,
MAMORU TABATA et al.: "Variation of
Alkaloid Production in Nicotiana rustica Callus
Cultures"**

The file contains technical information
submitted after the application was filed and not
included in this specification

(73) Proprietor: **GALLAHER LIMITED
65 Kingsway
London WC2B 6TG (GB)**

(72) Inventor: **Smith, Harry
Southlands 11 West Leake Road
East Leake Loughborough, Leicestershire (GB)**
Inventor: **Pearson, David William
56 Selby Road
West Bridgeford, Nottingham (GB)**

(74) Representative: **Perry, Robert Edward et al,
GILL JENNINGS & EVERY 53-64 Chancery Lane
London WC2A 1HN (GB)**

(56) References cited:
**AGR. BIOL. CHEM. 37, (8), 1973, pages 1857—
1864, ISAMU SHIIO et al.: "Nicotine
Production by Tobacco Callus Tissues and
Effect of Plant Growth Regulators"
FERMENTATION TECHNOLOGY TODAY,
"Proceedings of the international fermentation
symposium", 19—25 March 1972, pages
689—695 K. KATO et al.: "Liquid Suspension
culture of tobacco cells"**

Courier Press, Leamington Spa, England.

Preparing nicotine by culturing a nicotiana strain

This invention relates to a process for preparing nicotine by *in vitro* cultivation of a variety of *Nicotiana tabacum* or *Nicotiana rustica* in a nutrient culture medium.

Work which has hitherto been published on the cultivation of *Nicotiana tabacum* varieties implies that some, but by no means all, of the tested varieties contain low levels of nicotine. Much of the work has been done in conjunction with observations on growth using batches of callus culture. Cultivation in suspension cultures is described in Nature, Vol. 201 (1964), pp. 614 and in Biochem. Physiol. Pflanzen, 162, 513 (1971), pp. 503—513. In the former reference, it is stated that "the highest nicotine levels were found at the end of the growth". A conclusion of the latter reference is that callus cultures start to produce alkaloids (including nicotine) after the addition of cycloheximide (which inhibits growth), whereas, in suspension cultures, alkaloid production is dependent on the conditions of growth. It appears that alkaloid production was stimulated during sub-maximal growth, alkaloids accumulating during the initial phase of growth, e.g. within a period of five days. It was observed after the initial phase that the level of nicotine remained substantially constant. Another method of regulating growth discussed in the second reference, in order to achieve greater nicotine production, is by using the medium proposed in Ann. Sci. Nat. Biol. Veg. *14*, 1 (1953) by R. Heller.

It appears generally to be the case that nicotine production is at a maximum under conditions of sub-maximal growth but hitherto there has been no disclosure of how cultivation of *Nicotiana* varieties might be made a commercial proposition. The effect of hormone levels has been observed by Shiio *et al*, Agr. Biol. Chem. 37 (8), pp. 1857—1864, and Ohta *et al*, Agr. Biol. Chem. *42* (6), (1978), pp. 1245—1251, using callus cultures. The reported results, particularly of the former reference, are extremely difficult to interpret but, in general, there is no certainty that results achieved with callus cultures can be reproduced with suspension cultures. Callus cultures consist of large cell agglomerates, and nicotine may be produced even when the conditions are suitable for maximal growth, because cells within agglomerates are not growing. Indeed, one of the foremost workers in this field, Tabata, reported in "Frontiers of Plant Tissue Culture" (1978) ed. Thorpe, Proc. 4th Int. Cong, Plant Tissue and Cell Culture, that nicotine production may be up to 40% less in suspension culture than in callus culture.

In suspension culture, it is likely that an optimal growth medium will stimulate cell growth and inhibit nicotine production in a substantially uniform manner. Shiio *et al* and Ohta *et al* both used auxins in their cultivation media, and these hormones, at high concentration levels, tend to reduce cell agglomeration in liquid cultures but also to inhibit nicotine production. In particular, when Shiio *et al* grew *Nicotiana tabacum* var. Bright Yellow on a medium containing 1-naphthaleneacetic acid, they found that kinetin inhibited both growth and nicotine production.

British Patent Specification No. 1,387,821 discloses the production of a plant metabolite by a process which comprises a growth phase without cell differentiation, a phase in which the undifferentiated cells are cultivated, with differentiation, and a final phase in which the differentiated cells are cultivated. The growth phase may be conducted in a nutrient medium containing an auxin and a cytokinin, under maximal growth conditions. The second phase may be conducted in a fresh nutrient medium containing a lesser amount of auxin. Examples 27 and 28 disclose the use of *Nicotiana tabacum* in callus culture.

According to the present invention, a process for producing nicotine comprises cultivating a strain of *Nicotiana tabacum* or *Nicotiana rustica* in suspension culture under maximal growth conditions and, at or towards the end of the maximal growth phase, resuspending the strain in a fresh medium and cultivating the strain under sub-maximal growth conditions which do not inhibit nicotine production and in the presence of an auxin and a cytokinin. Thus it has been found that conditions can be achieved under which good nicotine yields can be obtained when both hormones are present. The nicotine can then be isolated from the system, i.e. from the cells and/or from the culture medium.

Suitable auxins are indoleacetic acid (IAA), 1-naphthaleneacetic acid (NAA) and 2,4-(dichlorophenoxy)acetic acid. NAA is the preferred auxin because it is heat-stable and can be sterilised by autoclaving. The preferred cytokinin is kinetin. As is known, the respective functions of auxins and cytokinins are on cell wall plasticity and cell expansion, and on cell division.

For any particular *Nicotiana* strain and any given growth nutrient medium, it is usually or invariably found that there are three phases: (1) an initial or lag phase during which there is little increase in fresh weight, although cell numbers may increase substantially; (2) a growth phase; and (3) a final phase in which there is little growth as the maximum has almost or completely been reached. It has now been found that, if a given Nicotiana strain in culture is removed from the medium at or towards the end of the growth phase (the second of the three phases discussed above) and suspended in fresh medium, the three phases can occur again.

As is well documented, it is possible to determine conditions of sub-maximal growth and high nicotine production for a given *Nicotiana* strain, and we find that it is possible to prepare a suspension culture of cells of that variety in a suitable medium and allow it to reach a high level of nicotine production, per unit biomass. The highest nicotine production per unit biomass may not occur until after, say, 7 days under such conditions. Prior to the sub-maximal growth stage, the maximal growth conditions produce the necessary biomass.

There are a number of ways in which conditions can be controlled in order to achieve sub-maximal growth when it is desired to start nicotine production. One is to introduce a growth inhibitor and another is to limit growth nutrients, i.e. to reduce or omit certain nutrients from the medium which should normally contain assimilable sources of carbon (such as sucrose or molasses) and nitrogen and other nutrients and vitamins. A particularly suitable medium for use in this invention is based on that proposed by Murashige and Skoog in Physiologia P1. *15* (1962), 473—497), which has been widely used for culturing *Nicotiana tabacum* varieties. The use of an increased level of thiamine, as recommended by Linsmaier and Skoog in Physiologia P1. *19* (1965), pp. 100—127, is also very satisfactory. The contents of such media can be controlled as desired in order to achieve sub-maximal growth.

Alternatively, growth may be inhibited by introducing a protein synthesis inhibitor such as cycloheximide, although this is less desirable as some proteins can be a desirable by-product of the process of this invention. A particularly preferred method of inhibiting growth and thereby increasing nicotine production is to control the hormone level.

The level of such hormones is critical and should not exceed 10 $\mu$M for, say, NAA, and 10 $\mu$M for, say, kinetin. In general, a concentration of less than these maximum amounts down to about 0.1 $\mu$M for the auxin and down to 0.01 $\mu$M for the cytokinin is suitable, preferred ranges being from 0.5 to 5 $\mu$M for the auxin and from 0.05 to 5 $\mu$M for the cytokinin. The preferred ranges for 2,4-D and analogous auxins may be a factor of 10 lower than for NAA.

It is often preferable to conduct the culture in the dark, e.g. in the substantial absence of visible light or under low intensity light. In this way, higher nicotine yields can be obtained than in light.

It is usually preferred to use a *Nicotiana tabacum*, rather than a *Nicotiana rustica*, strain. Particularly suitable *Nicotiana tabacum* varieties are Maryland Mammoth, Speight G 28 and, especially, N.C. 2512, although the Bright Yellow and Virginica varieties can also be used.

When it is desired to produce isotopically labelled, e.g. radioactive (e.g. $C^{14}$ or $N^{15}$ labelled), nicotine, then this may be achieved by including suitably labelled precursors in the medium, e.g. if all the $NH_4$ and $NO_3$ components in the medium are $N^{15}$-labelled.

Suspension cultures are obtained by transferring several grams of soft, loosely packed callus tissue into a liquid medium, followed by continuous gentle agitation. Although many tissues of the appropriate plant can be used as a source of callus, easily sterilised and relatively undifferentiated tissues are the most suitable. Consequently, cultures are usually derived either from stem pith or from sterile young seedlings. As the culture grows, single cells and small clumps of cells break away from the callus and become dispersed in the medium. After 1 to 3 weeks, depending on the growth rate of the culture, the suspended tissues are separated from the larger callus lumps by filtration or sedimentation, and can be subcultured by the addition of fresh medium, e.g. at 1:7 dilution.

The process of the invention can be adapted to operate more continuously than for batch cultures. For example, cells may be grown under maximal growth conditions in a first vessel into which fresh medium is continuously or intermittently introduced. The cells in the substantially exhausted medium are decanted or flow from this first vessel and are allowed to age in one or, preferably, a series of second vessels which overflow from one to the next as medium is introduced into the first vessel. The overflow of aged cells from the or the final second vessel is then diluted, in a third vessel or series of vessels, e.g. at 1:10 dilution, with a medium in which the cells grow, in a second growth phase, under sub-maximal growth conditions in the presence of the essential hormones, according to the invention. Nicotine can then be isolated from the system in the third vessel or vessels. It will be appreciated that the maximal and sub-maximal growth media may contain the same hormones, but at different concentrations.

## Claims

1. A process for producing nicotine, which comprises cultivating a strain of *Nicotiana tabacum* or *Nicotiana rustica* in suspension culture under maximal growth conditions and, at or towards the end of the maximal growth phase, resuspending the strain in a fresh medium and cultivating the strain under sub-maximal growth conditions which do not inhibit nicotine production and in the presence of an auxin and a cytokinin.

2. A process according to claim 1 wherein the auxin is indoleacetic acid, 1-naphthaleneacetic acid or 2,4-dichlorophenoxyacetic acid.

3. A process according to claim 2 wherein the auxin is 1-naphthaleneacetic acid.

4. A process according to claim 3 wherein the concentration of 1-naphthaleneacetic acid is from 0.5 to 5 $\mu$M.

5. A process according to any preceding claim wherein the cytokinin is kinetin.

6. A process according to claim 5 wherein the concentration of kinetin is from 0.5 to 5 $\mu$M.

7. A process according to any preceding claim wherein the strain is *Nicotiana tabacum* var Maryland Mammoth, *Nicotiana tabacum* var Speight G 28 or *Nicotiana tabacum* var N.C. 2512.

## Revendications

1. Un procédé pour produire de la nicotine qui comprend la culture d'une souche de *Nicotiana tabacum* ou de *Nicotiana rustica* en culture en suspension dans des conditions de croissance maximale et, à la fin ou vers la fin de la phase de croissance maximale, la remise en suspension de la souche dans un milieu frais et la culture de la souche dans des conditions de croissance submaximale qui n'inhibent pas la production de nicotine et en présence d'une auxine et d'une cytokinine.

2. Une procédé selon la revendication 1, dans lequel l'auxine est l'acide indole-acétique, l'acide naphtalène-1 acétique ou l'acide dichloro-2,4 phénoxyacétique.

3. Un procédé selon la revendication 2, dans lequel l'auxine est l'acide naphtalène-1 acétique.

4. Un procédé selon la revendication 3, dans lequel la concentration de l'acide naphtalène-1 acétique est de 0,5 à 5 $\mu$M.

5. Un procédé selon l'une quelconque des revendications précédentes, dans lequel la cytokinine est la kinétine.

6. Un procédé selon la revendication 5, dans lequel la concentration de la kinétine et de 0,5 à 5 $\mu$M.

7. Un procédé selon l'une quelconque des revendications précédentes, dans lequel la souche est *Nicotiana tabacum* var. Maryland Mammoth, *Nicotiana tabacum* var. Speight G 28 ou *Nicotiana tabacum* var. N.C. 2512.

## Patentansprüche

1. Ein Verfahren zur Herstellung von Nikotin, welches die Kultivierung eines Stammes von *Nicotiana tabacum* oder *Nicotiana rustica* in einer Suspensionskultur unter Bedingungen des maximalen Wachstums, am Ende oder gegen Ende der maximalen Wachstumsphase, ein Resuspendieren des Stammes in einem frischen Medium und ein Kultivieren des Stammes unter submaximalen Wachstumsbedingungen, welche die Nikotinbildung nicht inhibieren, und in Gegenwart eines Auxins und eines Cytokinins umfaßt.

2. Ein Verfahren nach Anspruch 1, worin das Auxin Indolessigsäure, 1-Naphthalinessigsäure oder 2,4-Dichlorphenoxyessigsäure ist.

3. Ein Verfahren nach Anspruch 2, worin das Auxin 1-Naphthalinessigsäure ist.

4. Ein Verfahren nach Anspruch 3, worin die Konzentration von 1-Naphthalinessigsäure von 0,5 bis 5 $\mu$Mol beträgt.

5. Ein Verfahren nach einem der vorstehenden Ansprüche, worin das Cytokinin Kinetin ist.

6. Ein Verfahren nach Anspruch 5, worin die Konzentration von Kinetin von 0,5 bis 5 $\mu$Mol beträgt.

7. Ein Verfahren nach einem der vorstehenden Ansprüche, worin der Stamm *Nicotiana tabacum* Var. Maryland Mammoth, *Nicotiana tabacum* Var. Speight G28 oder *Nicotiana tabacum* Var. N.C. 2512 ist.